# EUROPEAN PATENT APPLICATION

(11) **EP 3 733 707 A1**
(43) Date of publication of application: **04.11.2020**
(21) Application number: 19172053.1
(22) Date of filing: 30.04.2019
(51) Int. Cl.: C07K 16/28, C07K 14/705, A61K 35/17, A61P 35/00

(54) **CAR T-CELLS TARGETING BCMA AND USES THEREOF**

(71) Applicant: Celyad S.A., 1435 Mont-Saint-Guibert (BE)
(72) Inventor: The designation of the inventor has not yet been filed
(74) Representative: Goodacre, Jonathan David

(57) **Abstract**

The present application relates to the field of immunotherapy, more particularly to the field of chimeric antigen receptors (CARs). Here, CARs are proposed that are directed against B-cell Maturation Antigen (BCMA, also known as CD269). Also proposed are polynucleotides, vectors encoding the transmembrane polypeptide chains and cells expressing such CARs. These cells are particularly suitable for use in immunotherapy, and strategies to treat diseases such as cancer using these cells are also provided. The engineered immune cells, such as T-cells or natural killer (NK) cells, expressing such CARs are particularly suitable for treating lymphomas, multiple myeloma and leukemia.

## Description

### Field of the invention

The present application relates to the field of immunotherapy, more particularly to the field of chimeric antigen receptors (CARs). Here, CARs are proposed that are directed against B-cell Maturation Antigen (BCMA, also known as CD269). Also proposed are polynucleotides, vectors encoding the transmembrane polypeptide chains and cells expressing such CARs. These cells are particularly suitable for use in immunotherapy, and strategies to treat diseases such as cancer using these cells are also provided. The engineered immune cells, such as T-cells or natural killer (NK) cells, expressing such CARs are particularly suitable for treating lymphomas, multiple myeloma and leukemia.

### Background

The recent FDA approval of the first two CAR-T therapies, both directed against the B cell antigen CD19, has led to an ever-increasing interest in the CAR-T field. There is a continuous need for improvement of CAR design and novel targets. One of the most promising targets is another B cell antigen, called B-cell maturation antigen (BCMA, also known as TNFRSF17). BCMA is a tumor necrosis family receptor (TNFR) member expressed in cells of the B-cell lineage. BCMA expression is the highest on terminally differentiated B cells. BCMA is involved in mediating the survival of plasma cells for maintaining long-term humoral immunity. The expression of BCMA has been recently linked to a number of cancers, autoimmune disorders, and infectious diseases. Cancers with increased expression of BCMA include some hematological cancers, such as multiple myeloma, Hodgkin's and non-Hodgkin's lymphoma, various leukemias, and glioblastoma. Particularly in multiple myeloma, BCMA is considered a promising target, and several targeted therapies (including antibody-based therapies, ADC-based therapies and CAR-T based therapies) are under development.

Multiple myeloma (MM) is the second most common hematological malignancy and constitutes 2% of all cancer deaths. MM is a heterogeneous disease and caused by mostly by chromosome translocations inter alia t(I 1 ; 14),t(4; 14),t(8;14),del(13),del(17) (Drach et al., (1998) Blood 92(3): 802-809; Gertz et al., (2005) Blood 106(8):2837-2840; Facon et al., (2001) Blood 97(6): 1566-1571). MM-affected patients may experience a variety of disease-related symptoms due to, bone marrow infiltration, bone destruction, renal failure, immunodeficiency, and the psychosocial burden of a cancer diagnosis. The current 5-year survival rate for MM is approximately 50% highlighting that MM is a difficult-to-treat disease where there are currently no curative options.

While there already are numerous BCMA-targeted chimeric antigen receptor (CAR) T cells in development, each of them demonstrates a different efficacy and safety profile, and each uses a different construct. There is no link between the antibody binding efficacy and the therapeutic efficacy of the CAR. Although initial results and response rates of BCMA CAR-T therapies have generated excitement, it becomes ever more obvious that all of the BCMA CARs tested so far have a problem with durability of response and remission, and none of the BCMA CARs are close to being approved. The current CAR-T regimens also come at a cost of frequent adverse events, including cytokine release syndrome and neurotoxicity.

Thus, there is a continued need for alternative BCMA CAR-T therapies that can improve durability of response, particularly CAR-T that are highly effective and have less side effects (e.g. by being more specific or by reaching the same efficacy at lower, less toxic dose levels).

### Summary

In the current application, we developed numerous BCMA CARs and compare them side by side. To this end, a common backbone was used, where only the BCMA binding moiety was altered. These different CARs were tested in a number of test set-ups (see Examples section), and one particular CAR clearly outperformed the others. Surprisingly, the binding moiety for this construct came from an antibody that has not been used in a CAR before and binds to a conformational epitope composed of residues in the β-hairpin (residues Y13-H19) and helix-loop-helix (residues L26, R27, and N31-L35) regions of BCMA. The binding moiety can also disrupt the APRIL and BAFF signaling pathways in plasma cells through steric occlusion and direct competition for the BCMA binding site. Interestingly, APRIL and BAFF can signal using other receptors, such as TACI and BAFF-R, and BCMA knock-out mice are still viable. Therefore, blocking the APRIL and BAFF activity through BCMA occlusion with this CAR construct may not be critically toxic for MM patients.

Accordingly, it is an object of the invention to provide CAR constructs and CAR-T cells that target BCMA and are suitable for therapy.

According to a first aspect, chimeric antigen receptors are provided herein comprising an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

According to further embodiments, the anti-BCMA binding domain further comprises a light chain CDR1 having the amino acid sequence of GGNNIGSKSVH (SEQ ID NO: 4), a light chain CDR2 having the amino acid sequence of DDSDRPS (SEQ ID NO: 5), and a light chain CDR3 having the amino acid sequence of QVWDSSSDHVV (SEQ ID NO: 6).

According to particular embodiments, the heavy chain comprises the amino acid sequence of QLQLQESGPGLVKPSETLSLTCTVSGGSISSGSYFWGWIRQPPGKGLEWIGSIYYSGITYYNPSLKSRVTISVDTSKNQ FSLKLSSVTAADTAVYYCARHDGAVAGLFDYWGQGTLVTVSS (SEQ ID NO: 7). According to particular embodiments, the light chain comprises the amino acid sequence of

SYVLTQPPSVSVAPGQTARITCGGNNIGSKSVHWYQQPPGQAPVVVVYDDSDRPSGIPERFSGSNSGNTATLTISR
VEAGDEAVYYCQVWDSSSDHVVFGGGTKLTVL (SEQ ID NO: 8).

The antibody sequences have first been described in WO2017031104, incorporated herein in its entirety. Both the epitope and the paratope of the antibody have been characterized, and mutant antibody sequences have been generated therein. There, it was shown that several mutated CDR sequences bound BCMA with similar affinity. For instance, in the heavy chain CDR1, in the sequence SGSYFWG (SEQ ID NO: 1), the first G amino acid (at position 2 of SEQ ID NO: 1) can be mutated to a S and/or the F residue (at position 5) can be mutated to a Y while retaining or improving binding. Thus, CARs comprising the sequences SSSYFWG (SEQ ID NO: 9), SGSYYWG (SEQ ID NO: 10) and SSSYYWG (SEQ ID NO: 11) in heavy chain CDR1 are also provided herein. Particularly sequences with the G to S substitution (SEQ ID NO: 9 and 11) are envisaged. Further, in the heavy chain CDR2, in the sequence SIYYSGITYYNPSLKS (SEQ ID NO: 2), the second I amino acid (at position 7 of SEQ ID NO: 2) can be mutated to a S while retaining or improving binding. Thus, CARs comprising the sequences SIYYSGSTYYNPSLKS (SEQ ID NO: 12) in heavy chain CDR2 are also provided herein. Further, in the heavy chain CDR3, in the sequence HDGAVAGLFDY (SEQ ID NO: 3), the V amino acid (at position 5 of SEQ ID NO: 3) can be mutated to a T while retaining or improving binding. Thus, CARs comprising the sequences HDGATAGLFDY (SEQ ID NO: 13) in heavy chain CDR3 are also provided herein.

According to further embodiments, the signaling domain of the CAR molecules contains a signaling domain functional in immune cells. According to particular embodiments, the signaling domain comprises a signaling domain selected from the group consisting of a CD3 zeta domain, a Fc epsilon RI gamma domain and a CD3 epsilon domain.

The signaling domain may further contain accessory domains that reinforce or modify the signal. According to particular embodiments, the signaling domain further comprises a costimulatory domain. According to further particular embodiments, the costimulatory domain is selected from a CD28, 4-1BB, OX40, ICOS, DAP10, DAP12, CD27, and CD2 costimulatory domain.

According to a further aspect, CARs are not provided as proteins, but as nucleic acid molecules that encode these proteins (typically isolated nucleic acid molecules). Such nucleic acid molecules can then be expressed in suitable cells to generate the CARs (e.g. in T cells to generate CAR-T cells). These CARs encoded by nucleic acids may have all the features described herein.

Thus, nucleic acid molecules are provided encoding a CAR comprising an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

The anti-BCMA binding domain encoded by these nucleic acid molecules may further comprise a light chain CDR1 having the amino acid sequence of GGNNIGSKSVH (SEQ ID NO: 4), a light chain CDR2 having the amino acid sequence of DDSDRPS (SEQ ID NO: 5), and a light chain CDR3 having the amino acid sequence of QVWDSSSDHVV (SEQ ID NO: 6).

According to particular embodiments, the nucleic acid molecules encode a heavy chain which comprises the amino acid sequence of QLQLQESGPGLVKPSETLSLTCTVSGGSISSGSYFWGWIRQPPGKGLEWIGSIYYSGITYYNPSLKSRVTISVDTSKNQ FSLKLSSVTAADTAVYYCARHDGAVAGLFDYWGQGTLVTVSS (SEQ ID NO: 7). According to particular embodiments, the nucleic acid molecules encode a light chain which comprises the amino acid sequence of

SYVLTQPPSVSVAPGQTARITCGGNNIGSKSVHWYQQPPGQAPVVVVYDDSDRPSGIPERFSGSNSGNTATLTISR
VEAGDEAVYYCQVWDSSSDHVVFGGGTKLTVL (SEQ ID NO: 8).

Likewise, nucleic acids encoding CARs comprising the sequences SSSYFWG (SEQ ID NO: 9), SGSYYWG (SEQ ID NO: 10) and SSSYYWG (SEQ ID NO: 11) in heavy chain CDR1 are also provided herein. Particularly sequences with the G to S substitution (SEQ ID NO: 9 and 11) are envisaged. Further, in the heavy chain CDR2, in the sequence SIYYSGITYYNPSLKS (SEQ ID NO: 2), the second I amino acid (at position 7 of SEQ ID NO: 2) can be mutated to a S while retaining or improving binding. Thus, polynucleotides encoding CARs comprising the sequences SIYYSGSTYYNPSLKS (SEQ ID NO: 12) in heavy chain CDR2 are also provided herein. Further, in the heavy chain CDR3, in the sequence HDGAVAGLFDY (SEQ ID NO: 3), the V amino acid (at position 5 of SEQ ID NO: 3) can be mutated to a T while retaining or improving binding. Thus, nucleic acid molecules encoding CARs comprising the sequences HDGATAGLFDY (SEQ ID NO: 13) in heavy chain CDR3 are also provided herein.

According to further embodiments, the signaling domain of the CAR encoded by the nucleic acid molecules contains a signaling domain functional in immune cells. According to particular embodiments, the signaling domain comprises a signaling domain selected from the group consisting of a CD3 zeta domain, a Fc epsilon RI gamma domain and a CD3 epsilon domain.

The signaling domain encoded by the nucleic acid molecules may further contain accessory domains that reinforce or modify the signal. According to particular embodiments, the signaling domain further comprises a costimulatory domain. According to further particular embodiments, the costimulatory domain is selected from a CD28, 4-1BB, OX40, ICOS, DAP10, DAP12, CD27, and CD2 costimulatory domain.

Typically, the nucleic acid molecules will be provided in the form of a vector. Accordingly, vectors are provided comprising a nucleic acid molecule as described herein. Thus, provided are vectors comprising a nucleic acid molecule encoding a CAR comprising an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3). All of the metes and limitations of these CARs (and the specific domains) are as described herein.

In addition to the CAR, it is envisaged that the nucleic acid molecules and vectors may contain other features that enhance the therapeutic efficacy of the CAR. Examples of such features that can be encoded include, but are not limited to, checkpoint inhibitors, cytokines, chemokines, suicide genes, shRNA, antibodies, other CARs, anticalins, or the like.

According to a further aspect, the CARs are particularly useful when expressed in a cell, such as an immune cell. Accordingly, cells are provided comprising a CAR as described herein, that is: cells comprising a CAR containing an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

Also, cells are provided comprising a nucleic acid molecule as described herein, that is: cells comprising a nucleic acid molecule encoding a CAR comprising an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

Also, cells are provided comprising a vector as described herein, that is: cells comprising a vector containing a nucleic acid molecule encoding a CAR comprising an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

All of the features described for these CARs (and the specific domains) can thus also be provided in cells.

According to a further aspect, the cells described herein (i.e. containing an anti-BCMA CAR) are provided for use as a medicament. According to a further particular aspect, the cells described herein are provided for use in treating cancer. According to particular embodiments, the cancer is selected from leukemia, lymphoma, or multiple myeloma (MM). Most particularly, the cells are provided for use in treating multiple myeloma. According to alternative embodiments, the cells are provided for use in treating autoimmune disorders, as BCMA has been implicated in autoimmune disorders as well as B-lymphocyte malignancies. Examples of autoimmune diseases where BCMA inhibition has been proven useful include, but are not limited to, rheumatoid arthritis (RA), systemic lupus erythematosus (SLE) and multiple sclerosis (MS) (Hofmann et al., Front Immunol. 2018;9:835).

The cells containing a BCMA CAR can be autologous cells (the subject receives cells that originated from his or her body, but have been modified ex vivo) or can be allogeneic cells (the immune cells are derived from a donor, and have been modified ex vivo prior to administration to a subject that is not the donor).

This is equivalent as stating that methods of treating cancer are provided, comprising administering to a subject in need thereof a CAR containing an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

The cancer typically is selected from leukemia, lymphoma or MM. Most particularly, it is MM.

Alternatively, methods of treating an autoimmune disease are provided, comprising administering to a subject in need thereof a CAR containing an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

The autoimmune disease to be treated typically is selected from RA, SLE and MS.

In these methods of treatment, the CARs can further contain all of the features described for them or for specific domains. The cells typically are immune cells.

The methods for treatment can be autologous methods (the subject receives cells that originated from his or her body) or can be allogeneic methods (the cells are derived from a donor that is not the subject).

According to an alternative aspect, the nucleic acid molecules described herein (i.e. encoding an anti-BCMA CAR) are provided for use as a medicament. Typically, these nucleic acid molecules will then be used to transduce a cell so that it expresses a CAR. Although the cell will then typically be the agent that is administered to a subject in need thereof, nucleic acid molecules are typically easier to store or transport, and more amenable for use as a medicament. Also, although not particularly preferred, direct administration of nucleic acid molecules to a subject is envisaged herein as well. According to a further particular aspect, the nucleic acids described herein are provided for use in treating cancer. According to particular embodiments, the cancer is selected from leukemia, lymphoma, or multiple myeloma (MM). Most particularly, the nucleic acids are provided for use in treating multiple myeloma. According to alternative embodiments, the nucleic acid molecules are provided for use in treating autoimmune disorders. Exemplary autoimmune diseases include, but are not limited to, rheumatoid arthritis, systemic lupus erythematosus and multiple sclerosis.

Likewise, the vectors described herein (i.e. encoding an anti-BCMA CAR) are provided for use as a medicament. Typically, these vectors will then be used to transduce a cell so that it expresses a CAR. Although the cell will then typically be the agent that is administered to a subject in need thereof, vector molecules are typically easier to store or transport, and more amenable for use as a medicament. Also, although not particularly preferred, direct administration of vectors to a subject is envisaged herein as well. According to a further particular aspect, the vectors described herein are provided for use in treating cancer. According to particular embodiments, the cancer is selected from leukemia, lymphoma, or multiple myeloma (MM). Most particularly, the vectors are provided for use in treating multiple myeloma. According to alternative embodiments, the vectors are provided for use in treating autoimmune disorders. Exemplary autoimmune diseases include, but are not limited to, rheumatoid arthritis, systemic lupus erythematosus and multiple sclerosis.

This is equivalent as stating that methods of treating cancer are provided, comprising administering to a subject in need thereof a nucleic acid molecule encoding a CAR containing an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

Or, that methods of treating cancer are provided, comprising administering to a subject in need thereof a vector containing a nucleic acid molecule encoding a CAR containing an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

The cancer typically is selected from leukemia, lymphoma or MM. Most particularly, it is MM.

Alternatively, methods of treating an autoimmune disease are provided, comprising administering to a subject in need thereof a nucleic acid encoding a CAR containing an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

Or, methods of treating an autoimmune disease are provided, comprising administering to a subject in need thereof a vector containing a nucleic acid encoding a CAR containing an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

The autoimmune disease to be treated typically is selected from RA, SLE and MS.

In these methods of treatment, the CARs can further contain all of the features described for them or for specific domains.

However, most typically, the nucleic acid molecules and vectors will not be directly administered to a subject. Rather, they will be used in a method of adoptive cell transfer (ACT). ACT refers to the transfer of cells, most typically immune cells, into a patient. These cells may have originated from the patient (autologous therapy) or from another individual (allogeneic therapy). The goal of the therapy is to improve immune functionality and characteristics, and in cancer immunotherapy, to raise an immune response against the cancer. Although T cells are most often used for ACT, it is also applied using other immune cell types such as NK cells, lymphocytes (e.g. tumor-infiltrating lymphocytes (TILs)), dendritic cells and myeloid cells. Also stem cells such as induced pluripotent stem cells (iPSCs) may be used.

According to this aspect, cells will be provided (either obtained from a subject to be treated, or from a donor), and these cells will then be transduced, transfected or otherwise engineered with the nucleic acid molecules or vectors described herein. The resulting cells can then be administered to a subject (either the same subject, in case of autologous therapy, or a different subject, in case of allogeneic therapy).

Accordingly, methods of engineering a cell are provided, comprising:
- Providing cells derived from a subject
- Introducing in said cells a nucleic acid molecule encoding a CAR containing an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

Alternatively, methods of engineering a cell are provided, comprising:
- Providing cells derived from a subject
- Introducing in said cells a nucleic a vector containing a nucleic acid molecule encoding a CAR containing an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

According to particular embodiments, these methods are ex vivo or in vitro methods.

In these methods, the CARs can further contain all of the features described for them or for specific domains. The cells typically are immune cells. Most particularly, the cells are selected from the group of T cells, NK cells, NKT cells, lymphocytes, dendritic cells, myeloid cells, stem cells, progenitor cells or iPSCs.

When cells are being engineered for use in an allogeneic method, it is envisaged that the nucleic acid molecule or the vector will additionally contain features to prevent or reduce graft versus host disease, such as nucleic acid encoding inhibitors of TCR function (e.g. shRNA or TCR inhibitory molecules, as described in WO2013166051).

The methods may further comprise a step of administering the engineered cells to a subject in need thereof, making them methods of treatment. The methods for treatment can be autologous methods (the subject receives cells that originated from his or her body) or can be allogeneic methods (the cells are derived from a donor that is not the subject).

### Brief description of the Figures

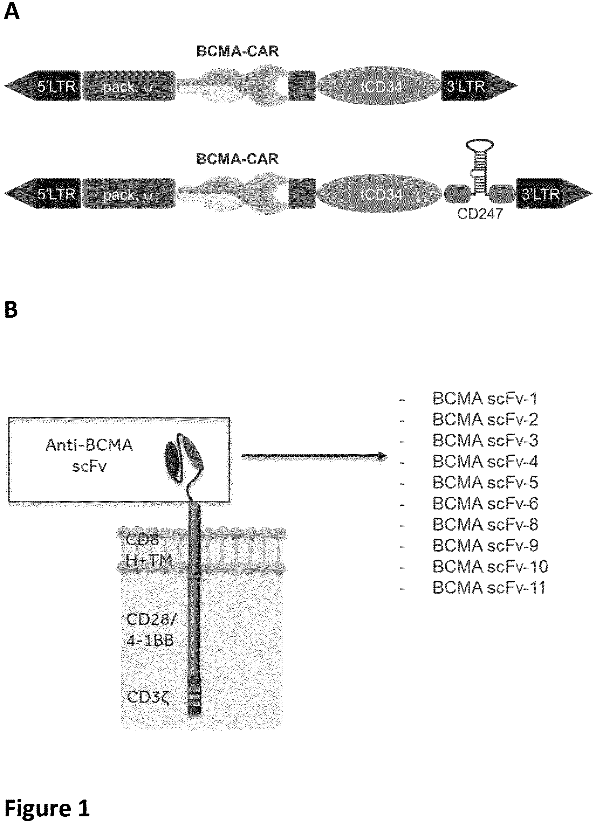

Figure 1. A: design of the BCMA CAR expression vector. LTR: Long terminal repeat; pack. Ψ: psi packaging signal; tCD34: truncated CD34, a marker protein; CD247: shRNA against CD247. The upper design is for autologous use, the lower design for allogeneic use, where the shRNA against CD247 inhibits TCR signaling. B: BCMA CAR protein lay-out. Anti-BCMA scFv: BCMA binding moiety; CD8 H+TM: hinge + transmembrane part of the molecule derived from CD8; CD28/4-1BB: costimulatory domains used; CD3ζ: CD3 zeta signaling domain.

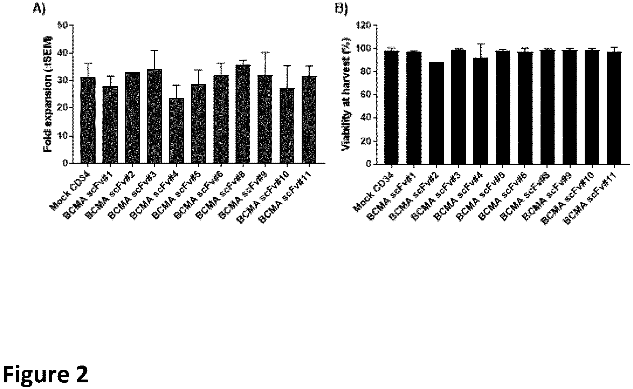

Figure 2. 11 different anti-BCMA scFv were tested in the BCMA CAR design of Figure 1. PBMCs from three different healthy donors were transduced with the indicated BCMA CAR construct or a Mock (tCD34) control vector. Fold cell expansion between day 4 to day 10 was compared (A). All constructs showed a similar fold expansion. Viability at harvest was comparable between cells transduced with the different BCMA CAR constructs (B).

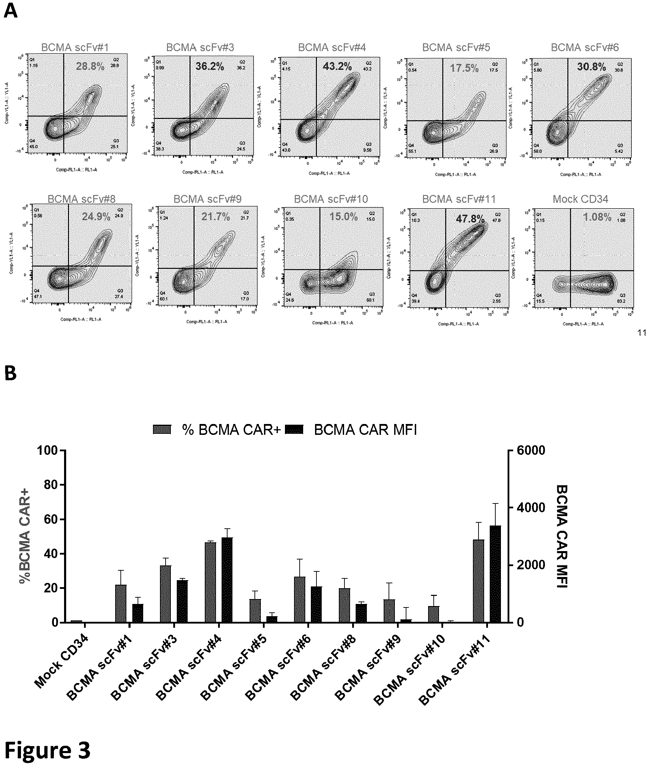

Figure 3. A. Cells transduced with the different BCMA-CAR constructs were stained with BCMA-Fc fusion protein. In a second step, cells were stained with PE-conjugated anti-Fc and an APC-conjugated anti-CD34 antibodies. Percentage of BCMA-CAR/tCD34 double positive cells is shown. B. Summary of the percentage BCMA-CAR positive cells (%) was assessed in total population of T cells. Median fluorescence intensity of BCMA-Fc staining is shown for CD34+ T cells.

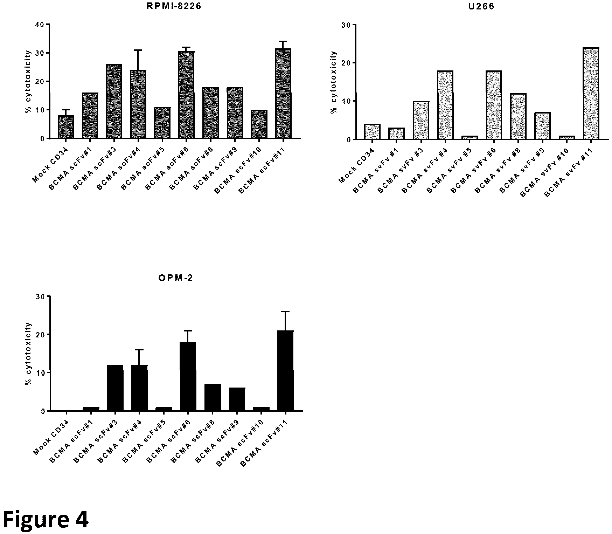

Figure 4. Cellular cytotoxicity of the different BCMA-CAR T cells was assessed by co-culturing the cells with different BCMA-positive cancer cell lines (RPMI-8226 (A), U266 (B), OPM-2 (C)). Lactate dehydrogenase (LDH) released into the media was used as a biomarker for cellular cytotoxicity and cytolysis.

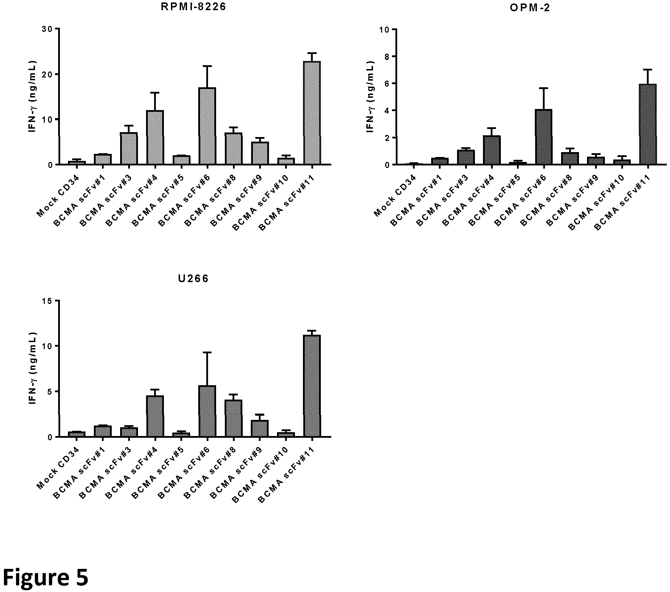

Figure 5. Different BCMA expressing cancer cell lines were co-cultured with Mock (tCD34), or BCMA-CAR expressing T cells. 24h after co-culture with different BCMA-positive cancer cell lines (RPMI-8226 (A), U266 (B), OPM-2 (C)), IFN-γ levels were measured in the supernatants.

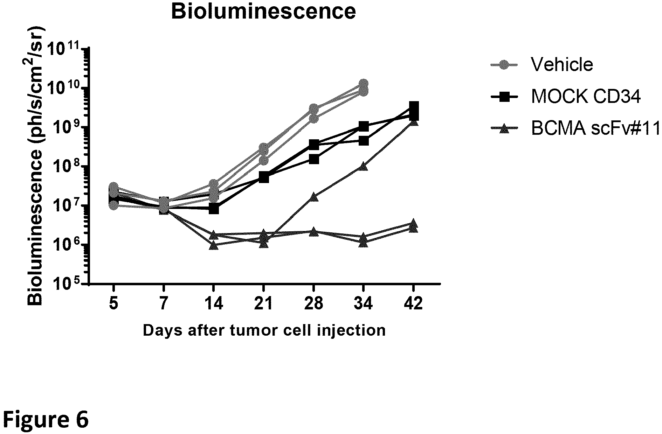

Figure 6. NSG mice were engrafted with KMS11-luc multiple myeloma cells. Mice were randomized following bioluminescence measurement at day 5 into three different groups. At day 6, animals were treated with vehicle control, tCD34 transduced T cells or T cells transduced with the selected BCMA-CAR. Bioluminescence values in photons per second that are leaving a square centimeter of tissue and radiating into a solid angle of one steradian are shown.

### Detailed description

### Definitions

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention.

Unless specifically defined herein, all terms used herein have the same meaning as they would to one skilled in the art of the present invention. Practitioners are particularly directed to Green and Sambrook, Molecular Cloning: A Laboratory Manual, 4th ed., Cold Spring Harbor Laboratory Press, , New York (2012); and Ausubel et al., Current Protocols in Molecular Biology (up to Supplement 114), John Wiley & Sons, New York (2016), for definitions and terms of the art. The definitions provided herein should not be construed to have a scope less than understood by a person of ordinary skill in the art.

A "chimeric antigen receptor" or "CAR" as used herein refers to a chimeric receptor (i.e. composed of parts from different sources) that has at least a binding moiety with a specificity for an antigen (which can e.g. be derived from an antibody, a receptor or its cognate ligand) and a signaling moiety that can transmit a signal in an immune cell (e.g. a CD3 zeta chain).

A "transmembrane domain" or "TM domain" as used herein is any membrane-spanning protein domain. Most typically, it is derived from a transmembrane protein. However, it can also be artificially designed. Transmembrane domains used herein will typically associate with other transmembrane domains, through charged and non-charged interactions.

The term "signaling domain" as used herein refers to a moiety that can transmit a signal in a cell, particularly in an immune cell. The signaling domain typically comprises a domain derived from a receptor that signals by itself in immune cells, such as the T Cell Receptor (TCR) complex or the Fc receptor. Additionally, it may contain a costimulatory domain (i.e. a domain derived from a receptor that is required in addition to the TCR to obtain full activation, or the full spectrum of the signal in case of inhibitory costimulatory domains, of T cells). The costimulatory domain can be from an activating costimulatory receptor or from an inhibitory costimulatory receptor.

The terms "B-cell maturation antigen", BCMA, CD269, or TNFRSF17 (UniProt Q02223; Gene ID: 608 in humans), as used herein refer to a member of the tumor necrosis receptor superfamily that is preferentially expressed in differentiated plasma cells (Laabi et al. (1992) EMBO J 11(11):3897-3904; Madry et al. (1998) Int Immunol 10(11):1693-1702). BCMA is a non-glycosylated type I transmembrane protein, which is involved in B cell maturation, growth and survival. BCMA is a receptor for two ligands of the TNF superfamily: APRIL (a proliferation-inducing ligand, CD256, TNFSF13), the high- affinity ligand to BCMA and the B cell activation factor BAFF (THANK, BlyS, B lymphocyte stimulator, TALL-1 and zTNF4), the low-affinity ligand to BCMA. APRIL and BAFF show structural similarity and overlapping yet distinct receptor binding specificity. The negative regulator TACI also binds to both BAFF and APRIL. An anti-BCMA binding domain is a domain that binds to the BCMA protein.

The term "immune cells" as used herein refers to cells that are part of the immune system (which can be either the adaptive or the innate immune system). Immune cells as used herein are typically immune cells that are manufactured for adoptive cell transfer (either autologous transfer or allogeneic transfer). Many different types of immune cells are used for adoptive therapy and thus are envisaged for use in the methods described herein. Examples of immune cells include, but are not limited to, T cells, NK cells, NKT cells, lymphocytes, dendritic cells, myeloid cells, stem cells, progenitor cells or iPSCs. The latter three are not immune cells as such, but can be used in adoptive cell transfer for immunotherapy (see e.g. Jiang et al., Cell Mol Immunol 2014; Themeli et al., Cell Stem Cell 2015). Typically, while the manufacturing starts with stem cells or iPSCs (or may even start with a dedifferentiation step from immune cells towards iPSCs), manufacturing will entail a step of differentiation to immune cells prior to administration. Stem cells, progenitor cells and iPSCs used in manufacturing of immune cells for adoptive transfer (i.e., stem cells, progenitor cells and iPSCs or their differentiated progeny that are transduced with a CAR as described herein) are considered as immune cells herein. According to particular embodiments, the stem cells envisaged in the methods do not involve a step of destruction of a human embryo.

Particularly envisaged immune cells include white blood cells (leukocytes), including lymphocytes, monocytes, macrophages and dendritic cells. Particularly envisaged lymphocytes include T cells, NK cells and B cells, most particularly envisaged are T cells. In the context of adoptive transfer, note that immune cells will typically be primary cells (i.e. cells isolated directly from human or animal tissue, and not or only briefly cultured), and not cell lines (i.e. cells that have been continually passaged over a long period of time and have acquired homogenous genotypic and phenotypic characteristics). According to specific embodiments, the immune cell is a primary cell. According to alternative specific embodiments, the immune cell is not a cell from a cell line.

"Antibody" refers to all isotypes of immunoglobulins (IgG, IgA, IgE, IgM, IgD, and IgY) including various monomelic, polymeric and chimeric forms, unless otherwise specified.

Specifically encompassed by the term "antibody" are polyclonal antibodies, monoclonal antibodies (mAbs), and antibody-like polypeptides, such as chimeric antibodies and humanized antibodies. "Antigen-binding fragments" are any proteinaceous structure that may exhibit binding affinity for a particular antigen. Antigen-binding fragments include those provided by any known technique, such as enzymatic cleavage, peptide synthesis, and recombinant techniques. Some antigen-binding fragments are composed of portions of intact antibodies that retain antigen-binding specificity of the parent antibody molecule. For example, antigen-binding fragments may comprise at least one variable region (either a heavy chain or light chain variable region) or one or more CDRs of an antibody known to bind a particular antigen. Examples of suitable antigen-binding fragments include, without limitation diabodies and single-chain molecules as well as Fab, F(ab')2, Fc, Fabc, and Fv molecules, single chain (Sc) antibodies, individual antibody light chains, individual antibody heavy chains, chimeric fusions between antibody chains or CDRs and other proteins, protein scaffolds, heavy chain monomers or dimers, light chain monomers or dimers, dimers consisting of one heavy and one light chain, a monovalent fragment consisting of the VL, VH, CL and CHI domains, or a monovalent antibody as described in WO2007059782, bivalent fragments comprising two Fab fragments linked by a disulfide bridge at the hinge region, a Fd fragment consisting essentially of the V.sub.H and C.sub.HI domains; a Fv fragment consisting essentially of the VL and VH domains of a single arm of an antibody, a dAb fragment (Ward et al., Nature 341, 544-546 (1989)), which consists essentially of a VH domain and also called domain antibodies (Holt et al; Trends Biotechnol. 2003 Nov.; 21(11):484-90); camelid or nanobodies (Revets et al; Expert Opin Biol Ther. 2005 Jan.; 5(1): 111-24); an isolated complementarity determining region (CDR), and the like. All antibody isotypes may be used to produce antigen-binding fragments. Additionally, antigen- binding fragments may include non-antibody proteinaceous frameworks that may successfully incorporate polypeptide segments in an orientation that confers affinity for a given antigen of interest, such as protein scaffolds. Antigen-binding fragments may be recombinantly produced or produced by enzymatic or chemical cleavage of intact antibodies. The phrase "an antibody or antigen-binding fragment thereof may be used to denote that a given antigen-binding fragment incorporates one or more amino acid segments of the antibody referred to in the phrase.

The term "epitope" means a protein determinant capable of specific binding to an antibody. Epitopes usually consist of surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents. The epitope may comprise amino acid residues directly involved in the binding and other amino acid residues, which are not directly involved in the binding, such as amino acid residues which are effectively blocked or covered by the specifically antigen binding peptide (in other words, the amino acid residue is within the footprint of the specifically antigen binding peptide).

"Specific binding" or "immunospecific binding" or derivatives thereof when used in the context of antibodies, or antibody fragments, represents binding via domains encoded by immunoglobulin genes or fragments of immunoglobulin genes to one or more epitopes of a protein of interest, without preferentially binding other molecules in a sample containing a mixed population of molecules. Typically, an antibody binds to a cognate antigen with a KD of less than about 1x10⁻⁸ M, as measured by a surface plasmon resonance assay or a cell binding assay. Phrases such as "[antigen] - specific" antibody (e.g., BCMA-specific antibody) are meant to convey that the recited antibody specifically binds the recited antigen.

"Isolated" as used herein means a biological component (such as a nucleic acid, peptide or protein) has been substantially separated, produced apart from, or purified away from other biological components of the organism in which the component naturally occurs, i.e., other chromosomal and extrachromosomal DNA and RNA, and proteins. Nucleic acids, peptides and proteins that have been "isolated" thus include nucleic acids and proteins purified by standard purification methods. "Isolated" nucleic acids, peptides and proteins can be part of a composition and still be isolated if such composition is not part of the native environment of the nucleic acid, peptide, or protein. The term also embraces nucleic acids, peptides and proteins prepared by recombinant expression in a host cell as well as chemically synthesized nucleic acids. An "isolated" antibody or antigen-binding fragment, as used herein, is intended to refer to an antibody or antigen- binding fragment which is substantially free of other antibodies or antigen-binding fragments having different antigenic specificities (for instance, an isolated antibody that specifically binds to BCMA is substantially free of antibodies that specifically bind antigens other than BCMA). An isolated antibody that specifically binds to an epitope, isoform or variant of BCMA may, however, have cross-reactivity to other related antigens, for instance from other species (such as BCMA species homologs).

The phrase "nucleic acid molecule" synonymously referred to as "nucleotides" or "nucleic acids" or "polynucleotide" refers to any polyribonucleotide or polydeoxyribonucleotide, which may be unmodified RNA or DNA or modified RNA or DNA. Nucleic acid molecules include, without limitation single- and double-stranded DNA, DNA that is a mixture of single- and double- stranded regions, single- and double-stranded RNA, and RNA that is mixture of single- and double-stranded regions, hybrid molecules comprising DNA and RNA that may be single- stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications may be made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short nucleic acid chains, often referred to as oligonucleotides.

A "vector" is a replicon, such as plasmid, phage, cosmid, or virus in which another nucleic acid segment may be operably inserted so as to bring about the replication or expression of the segment. A "clone" is a population of cells derived from a single cell or common ancestor by mitosis. A "cell line" is a clone of a primary cell that is capable of stable growth in vitro for many generations. In some examples provided herein, cells are transformed by transfecting the cells with DNA.

The terms "express" and "produce" are used synonymously herein, and refer to the biosynthesis of a gene product. These terms encompass the transcription of a gene into RNA. These terms also encompass translation of RNA into one or more polypeptides, and further encompass all naturally occurring post-transcriptional and post-translational modifications.

The term "subject" refers to human and non-human animals, including all vertebrates, e.g., mammals and non-mammals, such as non-human primates, mice, rabbits, sheep, dogs, cats, horses, cows, chickens, amphibians, and reptiles. In most particular embodiments of the described methods, the subject is a human.

The terms "treating" or "treatment" refer to any success or indicia of success in the attenuation or amelioration of an injury, pathology or condition, including any objective or subjective parameter such as abatement, remission, diminishing of symptoms or making the condition more tolerable to the patient, slowing in the rate of degeneration or decline, making the final point of degeneration less debilitating, improving a subject's physical or mental well-being, or prolonging the length of survival. The treatment may be assessed by objective or subjective parameters; including the results of a physical examination, neurological examination, or psychiatric evaluations.

An "effective amount" or "therapeutically effective amount" refers to an amount effective, at dosages and for periods of time necessary, to achieve a desired therapeutic result. A therapeutically effective amount of a therapeutic, such as the transformed immune cells described herein, may vary according to factors such as the disease state, age, sex, and weight of the individual, and the ability of the therapeutic (such as the cells) to elicit a desired response in the individual. A therapeutically effective amount is also one in which any toxic or detrimental effects of the therapeutic are outweighed by the therapeutically beneficial effects.

The phrase "graft versus host disease" or "GvHD" refers to a condition that might occur after an allogeneic transplant. In GvHD, the donated bone marrow, peripheral blood (stem) cells or other immune cells view the recipient's body as foreign, and the donated cells attack the body. As donor immunocompetent immune cells, such as T cells, are the main driver for GvHD, one strategy to prevent GvHD is by reducing (TCR-based) signaling in these immunocompetent cells, e.g. by directly or indirectly inhibiting the function of the TCR complex.

The present application is the first application to show a systematic side-by-side comparison of BCMA scFvs in a common CAR background. This allowed identification of a scFv that has not been used in a CAR before and that outperforms other scFvs in several experimental set-ups.

Accordingly, it is an object of the invention to provide chimeric antigen receptors comprising an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

According to further embodiments, the anti-BCMA binding domain further comprises a light chain CDR1 having the amino acid sequence of GGNNIGSKSVH (SEQ ID NO: 4), a light chain CDR2 having the amino acid sequence of DDSDRPS (SEQ ID NO: 5), and a light chain CDR3 having the amino acid sequence of QVWDSSSDHVV (SEQ ID NO: 6). As the light chain CDR1 is not actively involved in ligand binding, according to alternative embodiments, the anti-BCMA binding domain further comprises a light chain CDR2 having the amino acid sequence of DDSDRPS (SEQ ID NO: 5), and a light chain CDR3 having the amino acid sequence of QVWDSSSDHVV (SEQ ID NO: 6).

According to particular embodiments, the heavy chain comprises the amino acid sequence of QLQLQESGPGLVKPSETLSLTCTVSGGSISSGSYFWGWIRQPPGKGLEWIGSIYYSGITYYNPSLKSRVTISVDTSKNQ FSLKLSSVTAADTAVYYCARHDGAVAGLFDYWGQGTLVTVSS (SEQ ID NO: 7). According to particular embodiments, the light chain comprises the amino acid sequence of

SYVLTQPPSVSVAPGQTARITCGGNNIGSKSVHWYQQPPGQAPVVVVYDDSDRPSGIPERFSGSNSGNTATLTISR
VEAGDEAVYYCQVWDSSSDHVVFGGGTKLTVL (SEQ ID NO: 8).

The antibody sequences have first been described in WO2017031104, incorporated herein in its entirety. As described in Example 2 therein, a human immunoglobulin transgenic rat strain (OmniRat ®; OMT, Inc.) was used to develop human BCMA monoclonal antibody expressing hybridoma cells. When immunized with recombinant human BCMA (rhBCMA), this transgenic rat produces human IgG antibodies specific to human BCMA. Antibodies generated thus were purified and characterized, and the antibody derived from the M2 hybridoma-named BCMB69- was selected as the best hit. This antibody comprises SEQ ID NO: 7 and 8 as its heavy and light chains, respectively. After crystallization, the epitope, paratope and interactions were determined (Example 6 in WO2017031104). The antibody recognizes a conformational epitope composed of residues in the β-hairpin (residues Y13-H19) and helix-loop-helix (residues L26, R27, and N31-L35) regions of BCMA. Its epitope comprises an area of about 830 A² on BCMA and contains the ligand-binding DXL motif (residues D15-L18 in the type I turn of the β-hairpin), which protrudes into a shallow cavity lined by the antibody complementarity determining regions (CDRs). Leucine 17, at the tip of the DXL turn, is completely buried in the antibody cavity and has extensive interactions with the antibody. Another prevalent epitope residue is Arg27, which makes several hydrogen bond contacts with the heavy chain CDRs.

The antibody paratope is composed of residues from all CDRs except CDR-L1. Thus, according to some embodiments, the sequence of CDR1 in the light chain can be varied without impacting the properties of the antibody. The heavy chain has twice the number of contacts with BCMA compared to the light chain, and 40% of total contacts are made by CDR-H3.

Thus, both the epitope and the paratope of the antibody have been characterized, and mutant antibody sequences have been generated therein. There, it was shown that several mutated CDR sequences bound BCMA with similar affinity. For instance, in the heavy chain CDR1, in the sequence SGSYFWG (SEQ ID NO: 1), the first G amino acid (at position 2 of SEQ ID NO: 1) can be mutated to a S and/or the F residue (at position 5) can be mutated to a Y while retaining or improving binding. Thus, CARs comprising the sequences SSSYFWG (SEQ ID NO: 9), SGSYYWG (SEQ ID NO: 10) and SSSYYWG (SEQ ID NO: 11) in heavy chain CDR1 are also provided herein. Particularly sequences with the G to S substitution (SEQ ID NO: 9 and 11) are envisaged. Further, in the heavy chain CDR2, in the sequence SIYYSGITYYNPSLKS (SEQ ID NO: 2), the second I amino acid (at position 7 of SEQ ID NO: 2) can be mutated to a S while retaining or improving binding. Thus, CARs comprising the sequences SIYYSGSTYYNPSLKS (SEQ ID NO: 12) in heavy chain CDR2 are also provided herein. Further, in the heavy chain CDR3, in the sequence HDGAVAGLFDY (SEQ ID NO: 3), the V amino acid (at position 5 of SEQ ID NO: 3) can be mutated to a T while retaining or improving binding. Thus, CARs comprising the sequences HDGATAGLFDY (SEQ ID NO: 13) in heavy chain CDR3 are also provided herein.

According to further embodiments, the signaling domain of the CAR molecules contains a signaling domain functional in immune cells. According to particular embodiments, the signaling domain comprises a signaling domain selected from the group consisting of a CD3 zeta domain, a Fc epsilon RI gamma domain and a CD3 epsilon domain.

The signaling domain may further contain accessory domains that reinforce or modify the signal. According to particular embodiments, the signaling domain further comprises a costimulatory domain. According to further particular embodiments, the costimulatory domain is selected from a CD28, 4-1BB, OX40, ICOS, DAP10, DAP12, CD27, and CD2 costimulatory domain.

According to a further aspect, CARs are not provided as proteins, but as nucleic acid molecules that encode these proteins (typically isolated nucleic acid molecules). Such nucleic acid molecules can then be expressed in suitable cells to generate the CARs (e.g. in T cells to generate CAR-T cells). These CARs encoded by nucleic acids may have all the features described herein.

Thus, nucleic acid molecules are provided encoding a CAR comprising an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

The anti-BCMA binding domain encoded by these nucleic acid molecules may further comprise a light chain CDR1 having the amino acid sequence of GGNNIGSKSVH (SEQ ID NO: 4), a light chain CDR2 having the amino acid sequence of DDSDRPS (SEQ ID NO: 5), and a light chain CDR3 having the amino acid sequence of QVWDSSSDHVV (SEQ ID NO: 6).

According to particular embodiments, the nucleic acid molecules encode a heavy chain which comprises the amino acid sequence of QLQLQESGPGLVKPSETLSLTCTVSGGSISSGSYFWGWIRQPPGKGLEWIGSIYYSGITYYNPSLKSRVTISVDTSKNQ FSLKLSSVTAADTAVYYCARHDGAVAGLFDYWGQGTLVTVSS (SEQ ID NO: 7). According to particular embodiments, the nucleic acid molecules encode a light chain which comprises the amino acid sequence of 
SYVLTQPPSVSVAPGQTARITCGGNNIGSKSVHWYQQPPGQAPVVVVYDDSDRPSGIPERFSGSNSGNTATLTISR
VEAGDEAVYYCQVWDSSSDHVVFGGGTKLTVL (SEQ ID NO: 8).
 Likewise, nucleic acids encoding CARs comprising the sequences SSSYFWG (SEQ ID NO: 9), SGSYYWG (SEQ ID NO: 10) and SSSYYWG (SEQ ID NO: 11) in heavy chain CDR1 are also provided herein. Particularly sequences with the G to S substitution (SEQ ID NO: 9 and 11) are envisaged. Further, in the heavy chain CDR2, in the sequence SIYYSGITYYNPSLKS (SEQ ID NO: 2), the second I amino acid (at position 7 of SEQ ID NO: 2) can be mutated to a S while retaining or improving binding. Thus, polynucleotides encoding CARs comprising the sequences SIYYSGSTYYNPSLKS (SEQ ID NO: 12) in heavy chain CDR2 are also provided herein. Further, in the heavy chain CDR3, in the sequence HDGAVAGLFDY (SEQ ID NO: 3), the V amino acid (at position 5 of SEQ ID NO: 3) can be mutated to a T while retaining or improving binding. Thus, nucleic acid molecules encoding CARs comprising the sequences HDGATAGLFDY (SEQ ID NO: 13) in heavy chain CDR3 are also provided herein.

According to further embodiments, the signaling domain of the CAR encoded by the nucleic acid molecules contains a signaling domain functional in immune cells. According to particular embodiments, the signaling domain comprises a signaling domain selected from the group consisting of a CD3 zeta domain, a Fc epsilon RI gamma domain and a CD3 epsilon domain.

The signaling domain encoded by the nucleic acid molecules may further contain accessory domains that reinforce or modify the signal. According to particular embodiments, the signaling domain further comprises a costimulatory domain. According to further particular embodiments, the costimulatory domain is selected from a CD28, 4-1BB, OX40, ICOS, DAP10, DAP12, CD27, and CD2 costimulatory domain.

Typically, the nucleic acid molecules will be provided in the form of a vector. Accordingly, vectors are provided comprising a nucleic acid molecule as described herein. Thus, provided are vectors comprising a nucleic acid molecule encoding a CAR comprising an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3). All of the metes and limitations of these CARs (and the specific domains) are as described herein.

In addition to the CAR, it is envisaged that the nucleic acid molecules and vectors may contain other features that enhance the therapeutic efficacy of the CAR. Examples of such features that can be encoded include, but are not limited to, checkpoint inhibitors, cytokines, chemokines, suicide genes, shRNA, antibodies, other CARs, anticalins, or the like.

According to a further aspect, the CARs are particularly useful when expressed in a cell, such as an immune cell. Accordingly, cells are provided comprising a CAR as described herein, that is: cells comprising a CAR containing an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

Also, cells are provided comprising a nucleic acid molecule as described herein, that is: cells comprising a nucleic acid molecule encoding a CAR comprising an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

Also, cells are provided comprising a vector as described herein, that is: cells comprising a vector containing a nucleic acid molecule encoding a CAR comprising an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

All of the features described for these CARs (and the specific domains) can thus also be provided in cells.

According to a further aspect, the cells described herein (i.e. containing an anti-BCMA CAR) are provided for use as a medicament. According to a further particular aspect, the cells described herein are provided for use in treating cancer. According to particular embodiments, the cancer is selected from leukemia, lymphoma, or multiple myeloma (MM). Most particularly, the cells are provided for use in treating multiple myeloma. According to alternative embodiments, the cells are provided for use in treating autoimmune disorders, as BCMA has been implicated in autoimmune disorders as well as B-lymphocyte malignancies. Examples of autoimmune diseases where BCMA inhibition has been proven useful include, but are not limited to, rheumatoid arthritis (RA), systemic lupus erythematosus (SLE) and multiple sclerosis (MS) (Hofmann et al., Front Immunol. 2018;9:835).

The cells containing a BCMA CAR can be autologous cells (the subject receives cells that originated from his or her body, but have been modified ex vivo) or can be allogeneic cells (the immune cells are derived from a donor, and have been modified ex vivo prior to administration to a subject that is not the donor).

This is equivalent as stating that methods of treating cancer are provided, comprising administering to a subject in need thereof a CAR containing an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

The cancer typically is selected from leukemia, lymphoma or MM. Most particularly, it is MM. In principle, any BCMA-expressing cancer is amenable for treatment; BCMA is known to be a prominent antigen in MM.

Alternatively, methods of treating an autoimmune disease are provided, comprising administering to a subject in need thereof a CAR containing an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

The autoimmune disease to be treated typically is selected from RA, SLE and MS.

The CAR will typically be administered in an effective amount, i.e. an amount that results in the improvement of at least one symptom or parameter in the subject that receives the treatment. The methods may contain the further step of thereby improving at least one symptom or parameter of the disease.

In these methods of treatment, the CARs can further contain all of the features described for them or for specific domains.

The methods for treatment can be autologous methods (the subject receives cells that originated from his or her body) or can be allogeneic methods (the cells are derived from a donor that is not the subject).

According to an alternative aspect, the nucleic acid molecules described herein (i.e. encoding an anti-BCMA CAR) are provided for use as a medicament. Typically, these nucleic acid molecules will then be used to transduce a cell so that it expresses a CAR. Although the cell will then typically be the agent that is administered to a subject in need thereof, nucleic acid molecules are typically easier to store or transport, and more amenable for use as a medicament. Also, although not particularly preferred, direct administration of nucleic acid molecules to a subject is envisaged herein as well. According to a further particular aspect, the nucleic acids described herein are provided for use in treating cancer. According to particular embodiments, the cancer is selected from leukemia, lymphoma, or multiple myeloma (MM). Most particularly, the nucleic acids are provided for use in treating multiple myeloma. According to alternative embodiments, the nucleic acid molecules are provided for use in treating autoimmune disorders. Exemplary autoimmune diseases include, but are not limited to, rheumatoid arthritis, systemic lupus erythematosus and multiple sclerosis.

Likewise, the vectors described herein (i.e. encoding an anti-BCMA CAR) are provided for use as a medicament. Typically, these vectors will then be used to transduce a cell so that it expresses a CAR. Although the cell will then typically be the agent that is administered to a subject in need thereof, vector molecules are typically easier to store or transport, and more amenable for use as a medicament. Also, although not particularly preferred, direct administration of vectors to a subject is envisaged herein as well. According to a further particular aspect, the vectors described herein are provided for use in treating cancer. According to particular embodiments, the cancer is selected from leukemia, lymphoma, or multiple myeloma (MM). Most particularly, the vectors are provided for use in treating multiple myeloma. According to alternative embodiments, the vectors are provided for use in treating autoimmune disorders. Exemplary autoimmune diseases include, but are not limited to, rheumatoid arthritis, systemic lupus erythematosus and multiple sclerosis.

This is equivalent as stating that methods of treating cancer are provided, comprising administering to a subject in need thereof a nucleic acid molecule encoding a CAR containing an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

Or, that methods of treating cancer are provided, comprising administering to a subject in need thereof a vector containing a nucleic acid molecule encoding a CAR containing an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

The cancer typically is selected from leukemia, lymphoma or MM. Most particularly, it is MM.

Alternatively, methods of treating an autoimmune disease are provided, comprising administering to a subject in need thereof a nucleic acid encoding a CAR containing an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

Or, methods of treating an autoimmune disease are provided, comprising administering to a subject in need thereof a vector containing a nucleic acid encoding a CAR containing an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

The autoimmune disease to be treated typically is selected from RA, SLE and MS.

The nucleic acids or vectors will typically be administered in an effective amount, i.e. an amount that results in the improvement of at least one symptom or parameter in the subject that receives the treatment. The methods may contain the further step of thereby improving at least one symptom or parameter of the disease.

In these methods of treatment, the CARs can further contain all of the features described for them or for specific domains. The nucleic acids or vectors encoding the CARs described herein may contain further features to improve therapeutic efficacy. For instance, when provided for use in allogeneic methods, the nucleic acids/vectors typically will further comprise an inhibitor of the T cell receptor complex, to prevent GvHD. Other elements typically contained in nucleic acid or vector constructs include, but are not limited to, checkpoint inhibitors, cytokines, chemokines, and the like.

However, most typically, the nucleic acid molecules and vectors will not be directly administered to a subject. Rather, they will be used in a method of adoptive cell transfer (ACT). ACT refers to the transfer of cells, most typically immune cells, into a patient. These cells may have originated from the patient (autologous therapy) or from another individual (allogeneic therapy). The goal of the therapy is to improve immune functionality and characteristics, and in cancer immunotherapy, to raise an immune response against the cancer. Although T cells are most often used for ACT, it is also applied using other immune cell types such as NK cells, lymphocytes (e.g. tumor-infiltrating lymphocytes (TILs)), dendritic cells and myeloid cells. Also stem cells such as induced pluripotent stem cells (iPSCs) may be used.

According to this aspect, cells will be provided (either obtained from a subject to be treated, or from a donor), and these cells will then be transduced, transfected or otherwise engineered with the nucleic acid molecules or vectors described herein. The resulting cells can then be administered to a subject (either the same subject, in case of autologous therapy, or a different subject, in case of allogeneic therapy).

Accordingly, methods of engineering a cell are provided, comprising:
- Providing cells derived from a subject
- Introducing in said cells a nucleic acid molecule encoding a CAR containing an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

Alternatively, methods of engineering a cell are provided, comprising:
- Providing cells derived from a subject
- Introducing in said cells a vector containing a nucleic acid molecule encoding a CAR containing an anti-BCMA binding domain, a transmembrane domain, and an intracellular signaling domain, wherein said anti-BCMA binding domain comprises a heavy chain complementarity determining region 1 (CDR1) having the amino acid sequence of SGSYFWG (SEQ ID NO: 1), a heavy chain CDR2 having the amino acid sequence of SIYYSGITYYNPSLKS (SEQ ID NO: 2), and a heavy chain CDR3 having the amino acid sequence of HDGAVAGLFDY (SEQ ID NO: 3).

According to particular embodiments, these methods are ex vivo or in vitro methods.

In these methods, the CARs can further contain all of the features described for them or for specific domains. The cells typically are immune cells. Most particularly, the cells are selected from the group of T cells, NK cells, NKT cells, lymphocytes, dendritic cells, myeloid cells, stem cells, progenitor cells or iPSCs.

When cells are being engineered for use in an allogeneic method, it is envisaged that the nucleic acid molecule or the vector will additionally contain features to prevent or reduce graft versus host disease, such as nucleic acid encoding inhibitors of TCR function (e.g. shRNA or TCR inhibitory molecules, as described in WO2013166051).

The methods may further comprise a step of administering the engineered cells to a subject in need thereof, making them methods of treatment. The methods for treatment can be autologous methods (the subject receives cells that originated from his or her body) or can be allogeneic methods (the cells are derived from a donor that is not the subject).

The cells will typically be administered in an effective amount, i.e. an amount that results in the improvement of at least one symptom or parameter in the subject that receives the treatment. The methods may contain the further step of thereby improving at least one symptom or parameter of the disease.

It is to be understood that although particular embodiments, specific configurations as well as materials and/or molecules, have been discussed herein for cells and methods according to present invention, various changes or modifications in form and detail may be made without departing from the scope and spirit of this invention. The following examples are provided to better illustrate particular embodiments, and they should not be considered limiting the application. The application is limited only by the claims.

### Examples

### Example 1. Design of several BCMA CARs

To be able to compare different binding moieties for an improved BCMA CAR side by side, an identical backbone construct was used, where only the binding moiety of the BCMA-CAR was altered between the different constructs to be tested. This backbone construct is shown in Figure 1A, is based on a retroviral vector and further contains a marker. Further, for use in allogeneic setting, an identical construct was made that further contained shRNA against CD247 (Figure 1A, bottom). When expressed in cells, this shRNA interferes with the function of the T cell receptor complex and thus can prevent the development of graft versus host disease (GvHD) in allogeneic ACT.

The design of the BCMA CAR is shown in more detail in Figure 1B. Eleven different binding moieties were tested, but the hinge, transmembrane and CD3 zeta signaling domains were kept identical. Each construct was tested in a version with either a CD28 or a 4-1BB costimulatory domain. For the BCMA binding moieties (all anti-BCMA scFvs), some sequences were taken from BCMA CARs currently in development (as a benchmark), whereas other sequences were used from BCMA antibodies that have not been used in a CAR before. Examples of CAR sequences are those from WO2013154760, WO2015158671 and WO2016014789.

### Example 2. Properties of different CARs in vitro

To test whether all CARs could be successfully expressed, PBMCs from three different healthy donors were transduced with the indicated BCMA CAR construct or a Mock (tCD34) control vector. Fold cell expansion between day 4 to day 10 was compared (Figure 2A). All constructs showed a similar fold expansion. Viability at harvest was also comparable between cells transduced with the different BCMA CAR constructs (Figure 2B). Results from here onwards are done with 4-1BB based CARs.

Next, in order to assess the affinity with which the different scFvs bind to BCMA, cells transduced with the different BCMA-CAR constructs were stained with BCMA-Fc fusion protein. In a second step, cells were stained with PE-conjugated anti-Fc and an APC-conjugated anti-CD34 antibodies. Percentage of BCMA-CAR/tCD34 double positive cells is shown in Figure 3A.

Figure 3B shows the summary of the percentage BCMA-CAR positive cells (%), assessed in the total population of T cells. Median fluorescence intensity (MFI) of BCMA-Fc staining is also shown in Fig. 3B, for CD34+ T cells. Here, it can be seen that some scFvs have a better affinity for BCMA than others. The difference is not due to variation in expression levels, as there is no change in the tCD34 levels from the same vector. Thus, the affinity difference is for most part due to BCMA recognition and cannot be attributed to expression alone.

### Example 3. Activity against tumor cells

To further evaluate the activity of different anti-BCMA CARs, cellular toxicity of the different BCMA-CAR T cells was assessed by co-culturing the cells with different BCMA-positive cancer cell lines (RPMI-8226, U266, OPM-2). Lactate dehydrogenase (LDH) released into the media was used as a biomarker for cellular cytotoxicity and cytolysis. Results are shown in Figure 4.

Likewise, interferon gamma secretion was used to measure activity against cancer cells. To this end, different BCMA expressing cancer cell lines were co-cultured with Mock (tCD34), or BCMA-CAR expressing T cells. 24h after co-culture, IFN-γ levels were measured in the supernatants. Results are shown in Figure 5.

These experiments clearly show that BCMA scFv #11 outperforms the other scFvs tested: not only does it have high expression levels and affinity, it is also the CAR that has the highest activity against the three distinct cancer cell lines tested.

Remarkably, scFv #11 is derived from an antibody that has not before been used in a CAR, and in these assays thus outperforms several of the scFvs currently in clinical development. scFv#11 is a sequence that has been derived from WO2017031104, Anti-bcma antibodies, bispecific antigen binding molecules that bind bcma and cd3, and uses thereof, incorporated herein in its entirety. Briefly, the binding sequences of the antibody described therein have been used to create a scFv by fusing the variable regions of the heavy and light chains interspersed with a linker peptide.

### Example 4. In vivo activity against tumor

To check whether the superior performance of scFv #11 in *in vitro* assays also translates to in vivo anti-tumor efficacy, an experiment was performed in mice. NSG mice were engrafted with KMS11-luc multiple myeloma cells. Mice were randomized following bioluminescence measurement at day 5 into three different groups. At day 6, animals were treated with vehicle control, tCD34 transduced T cells or T cells transduced with the selected BCMA-CAR. Results are shown in Figure 6.

While in vehicle- and mock-treated animals, the tumor continues to grow, in mice treated with the selected BCMA CAR, the tumors shrink. In one of three animals, the tumor starts to regrow, while in two others, the bioluminescence appears to stay at a plateau level. This appears due to background luminescence, as these two animals appear to be tumor-free.

## Claims

1. A chimeric antigen receptor (CAR) comprising an anti-BCMA binding domain, a transmembrane domain, and an intracellular signaling domain, wherein said anti-BCMA binding domain comprises a heavy chain complementarity determining region 1 (CDR1) having the amino acid sequence of SEQ ID NO: 1, a heavy chain CDR2 having the amino acid sequence of SEQ ID NO: 2, and a heavy chain CDR3 having the amino acid sequence of SEQ ID NO: 3.

2. The CAR of claim 1, wherein said anti-BCMA binding domain further comprises a light chain CDR1 having the amino acid sequence of SEQ ID NO: 4, a light chain CDR2 having the amino acid sequence of SEQ ID NO: 5, and a light chain CDR3 having the amino acid sequence of SEQ ID NO: 6.

3. The CAR of any one of claim 1 or 2, wherein the heavy chain comprises the amino acid sequence of SEQ ID NO: 7.

4. The CAR of any one of claim 1 to 3, wherein the light chain comprises the amino acid sequence of SEQ ID NO: 8.

5. The CAR of any one of claim 1 to 4, wherein there is a G to S and/or a F to Y mutation in CDR1.

6. The CAR of any one of claim 1 to 5, wherein there is an I to S mutation at position 7 of CDR2.

7. The CAR of any one of claim 1 to 6, wherein there is an V to T mutation in CDR3.

8. The CAR of any one of claim 1 to 7, wherein the signaling domain comprises a signaling domain selected from the group consisting of a CD3 zeta domain, a Fc epsilon RI gamma domain and a CD3 epsilon domain.

9. The CAR of any one of claim 1 to 8, wherein the signaling domain further comprises a costimulatory domain selected from CD28, 4-1BB, OX40, ICOS, DAP10, DAP12, CD27, and CD2.

10. A nucleic acid molecule encoding a CAR according to any one of claims 1 to 9.

11. A vector comprising a nucleic acid molecule according to claim 10.

12. A cell comprising a CAR according to claim 1 to 9, nucleic acid molecule of claim 10 or vector of claim 11.

13. The cell of claim 12 or nucleic acid molecule of claim 10 for use as a medicament.

14. The cell of claim 12 or nucleic acid molecule of claim 10 for use in treating cancer.

15. The cell or nucleic acid molecule of claim 14, wherein the cancer is selected from leukemia, lymphoma, or multiple myeloma (MM).

16. A method of treating cancer in a subject in need thereof, comprising administering to said subject a cell comprising a CAR according to claim 1 to 9, nucleic acid molecule of claim 10 or vector of claim 11.

17. The method of claim 15, wherein the cancer is selected from leukemia, lymphoma, or multiple myeloma (MM).


SEQUENCE LISTING

<110> Celyad S.A.

<120> CAR T-cells targeting BCMA and uses thereof

<130> P50848EP/DJC/JG

<160> 13

<170> BiSSAP 1.3.6

<210> 1
<211> 7
<212> PRT
<213> Artificial Sequence

<220>
<223> Anti-BCMA binding domain heavy chain CDR1

<400> 1
Ser Gly Ser Tyr Phe Trp Gly
1               5

<210> 2
<211> 16
<212> PRT
<213> Artificial Sequence

<220>
<223> Anti-BCMA binding domain heavy chain CDR2

<400> 2
Ser Ile Tyr Tyr Ser Gly Ile Thr Tyr Tyr Asn Pro Ser Leu Lys Ser
1               5                   10                  15

<210> 3
<211> 11
<212> PRT
<213> Artificial Sequence

<220>
<223> Anti-BCMA binding domain heavy chain CDR3

<400> 3
His Asp Gly Ala Val Ala Gly Leu Phe Asp Tyr
1               5                   10

<210> 4
<211> 11
<212> PRT
<213> Artificial Sequence

<220>
<223> Anti-BCMA binding domain light chain CDR1

<400> 4

Gly Gly Asn Asn Ile Gly Ser Lys Ser Val His
1               5                   10

<210> 5
<211> 7
<212> PRT
<213> Artificial Sequence

<220>
<223> Anti-BCMA binding domain light chain CDR2

<400> 5
Asp Asp Ser Asp Arg Pro Ser
1               5

<210> 6
<211> 11
<212> PRT
<213> Artificial Sequence

<220>
<223> Anti-BCMA binding domain light chain CDR3

<400> 6
Gln Val Trp Asp Ser Ser Ser Asp His Val Val
1               5                   10

<210> 7
<211> 121
<212> PRT
<213> Artificial Sequence

<220>
<223> Anti-BCMA binding domain heavy chain

<400> 7
Gln Leu Gln Leu Gln Glu Ser Gly Pro Gly Leu Val Lys Pro Ser Glu
1               5                   10                  15
Thr Leu Ser Leu Thr Cys Thr Val Ser Gly Gly Ser Ile Ser Ser Gly
            20                  25                  30
Ser Tyr Phe Trp Gly Trp Ile Arg Gln Pro Pro Gly Lys Gly Leu Glu
        35                  40                  45
Trp Ile Gly Ser Ile Tyr Tyr Ser Gly Ile Thr Tyr Tyr Asn Pro Ser
    50                  55                  60
Leu Lys Ser Arg Val Thr Ile Ser Val Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg His Asp Gly Ala Val Ala Gly Leu Phe Asp Tyr Trp Gly
            100                 105                 110
Gln Gly Thr Leu Val Thr Val Ser Ser
        115                 120

<210> 8
<211> 108
<212> PRT
<213> Artificial Sequence

<220>

<223> Anti-BCMA binding domain light chain

<400> 8
Ser Tyr Val Leu Thr Gln Pro Pro Ser Val Ser Val Ala Pro Gly Gln
1               5                   10                  15
Thr Ala Arg Ile Thr Cys Gly Gly Asn Asn Ile Gly Ser Lys Ser Val
            20                  25                  30
His Trp Tyr Gln Gln Pro Pro Gly Gln Ala Pro Val Val Val Val Tyr
        35                  40                  45
Asp Asp Ser Asp Arg Pro Ser Gly Ile Pro Glu Arg Phe Ser Gly Ser
    50                  55                  60
Asn Ser Gly Asn Thr Ala Thr Leu Thr Ile Ser Arg Val Glu Ala Gly
65                  70                  75                  80
Asp Glu Ala Val Tyr Tyr Cys Gln Val Trp Asp Ser Ser Ser Asp His
                85                  90                  95
Val Val Phe Gly Gly Gly Thr Lys Leu Thr Val Leu
            100                 105

<210> 9
<211> 7
<212> PRT
<213> Artificial Sequence

<220>
<223> Anti-BCMA binding domain heavy chain CDR1

<400> 9
Ser Ser Ser Tyr Phe Trp Gly
1               5

<210> 10
<211> 7
<212> PRT
<213> Artificial Sequence

<220>
<223> Anti-BCMA binding domain heavy chain CDR1

<400> 10
Ser Gly Ser Tyr Tyr Trp Gly
1               5

<210> 11
<211> 7
<212> PRT
<213> Artificial Sequence

<220>
<223> Anti-BCMA binding domain heavy chain CDR1

<400> 11
Ser Ser Ser Tyr Tyr Trp Gly
1               5

<210> 12
<211> 16
<212> PRT
<213> Artificial Sequence

<220>
<223> Anti-BCMA binding domain heavy chain CDR2

<400> 12
Ser Ile Tyr Tyr Ser Gly Ser Thr Tyr Tyr Asn Pro Ser Leu Lys Ser
1               5                   10                  15

<210> 13
<211> 11
<212> PRT
<213> Artificial Sequence

<220>
<223> Anti-BCMA binding domain heavy chain CDR3

<400> 13
His Asp Gly Ala Thr Ala Gly Leu Phe Asp Tyr
1               5                   10